# EUROPEAN PATENT APPLICATION

(11) **EP 2 055 727 A1**
(43) Date of publication of application: **06.05.2009**
(21) Application number: 07301514.1
(22) Date of filing: 30.10.2007
(51) Int. Cl.: C08G 6/00, C03C 17/34, G01N 33/543, G01N 33/545

(54) **High-capacity nanoparticulate immobilization surface**

(71) Applicant: Corning Incorporated, Corning NY 14831 (US)
(72) Inventor: Deshayes, Sophie, 77515, Faremoutiers (FR); Henry, David, 91150, Morigny-Champigny (FR)
(74) Representative: Le Roux, Martine

(57) **Abstract**

Disclosed are articles comprising polyglutaraldehyde nanoparticulates modified substrates for use, for example, in binding biomolecules, and methods of making and using the modified substrates.

## Description

The present invention mainly deals with a method of making articles having a PGL (polyglutaraldehyde) nanoparticulate modified surface and with such articles.

### BACKGROUND

The disclosure relates to the field of biosensors for label-independent detection (LID). More particularly the disclosure relates to LID biosensor surface chemistry and to methods for making and use of a surface modified biosensor.

### SUMMARY

The disclosure provides polyglutaraldehyde (PGL) nanoparticulate modified substrates for binding biomolecules. The disclosure also provides methods of making and using the substrates.

### BRIEF DESCRIPTION OF THE DRAWINGS

Fig. 1A shows a schematic synthesis of polyglutaraldehyde (PGL) and Fig.1B a possible alternative structure of a PGL polymer, in embodiments of the disclosure.
Fig. 2 shows the UV spectrum of a water soluble polyglutaraldehyde compared to a monomeric glutaraldehyde, in embodiments of the disclosure.
Fig. 3 shows an exemplary FTIR spectrum of a water insoluble polyglutaraldehyde, in embodiments of the disclosure.
Fig. 4 shows Cy5-labeled streptavidin (Cy5-SA) immobilization capacity at three pH values on an exemplary PGL surface and compared with two reference aldehyde surfaces, in embodiments of the disclosure.
Fig. 5 shows a SEM micrograph of a deposited PGL colloid, in embodiments of the disclosure.
Fig. 6 shows exemplary fluorescent photographs and the corresponding RFU values that demonstrate the level of Cy5-SA immobilization on PGL coatings obtained with "poor" solvent, that is a weak or non-solvent for PGL, and a "good" solvent, that is a strong solvent for PGL, respectively, in embodiments of the disclosure.
Fig. 7 shows a Cy5-SA immobilization without NaCNBH₃ on a PGL coated Epic^{®} plate prepared with an exemplary PGL formulation, in embodiments of the disclosure.
Fig. 8 shows an SA immobilization with NaCNBH₃ on a PGL coated Epic^{®} plate prepared with an exemplary PGL formulation, in embodiments of the disclosure.
Fig. 9 shows binding results of 4-fluorescein-biotin on streptavidin immobilized using NaCNBH₃ on a PGL Epic^{®} coated plate prepared with a PGL formulation, in embodiments of the disclosure.
Fig. 10 shows average particle size and size distribution data obtained by dynamic light scattering (DLS) for a precipitated PGL nanoparticle dispersion of 0.25 wt % PGL in a DMSO/H₂O-0.1% SDS surfactant mixture, in embodiments of the disclosure.
Fig. 11 shows an exemplary SA immobilization using NaCNBH₃ on an Epic^{®} PGL coated plate using a PGL formulation, in embodiments of the disclosure.
Fig. 12 shows exemplary binding data of 4-fluorescein-biotin on streptavidin immobilized using NaCNBH₃ on PGL Epic^{®} coated plate, in embodiments of the disclosure.
Fig. 13 shows average particle size and size distribution data obtained by dynamic light scattering (DLS) for a precipitated PGL nanoparticle dispersion of a 0.05% wt PGL in DMSO/H₂O-0.05% SDS mixture, in embodiments of the disclosure.
Fig. 14 shows exemplary SA immobilization without NaCNBH₃ on a PGL coated plate prepared using the PGL formulation, in embodiments of the disclosure.
Figs. 15A-15D show the reproducibility of exemplary binding results for experiments of 4-fluorescein-biotin on SA immobilized without NaCNBH₃ on a PGL coated Epic^{®} plate prepared using the formulations of Examples 4A, 4B, and 4C, respectively, in embodiments of the disclosure.
Fig. 15E shows related exemplary binding results of 4-fluorescein-biotin on streptavidin immobilized with K₂HPO₄ on a PGL coated Epic^{®} plate prepared with a PGL formulation, in embodiments of the disclosure.

### DETAILED DESCRIPTION

Various embodiments of the disclosure will be described in detail with reference to drawings, if any. Reference to various embodiments does not limit the scope of the invention, which is limited only by the scope of the claims attached hereto. Additionally, any examples set forth in this specification are not intended to be limiting and merely set forth some of the many possible embodiments for the claimed invention.

### Definitions

"Attach," "attachment," "adhere," "adhered," "immobilized", or like terms generally refer to immobilizing or fixing for example, by any physical-chemical interaction between two or more components or compounds, for example, a protein or like synthetic or natural biological, a surface modifier substance, a compatibilizer, a cell, a ligand candidate compound, and like entities within the scope of the disclosure, such as to a surface, such as by physical absorption, chemical bonding, and like attachment interactions, or combinations thereof. Examples of attachment interactions can include, for example, covalent, electrostatic, ionic, hydrogen, hydrophobic bonding, and like interactions, or combinations thereof. The type and extent of physical-chemical interaction that can be formed will vary depending upon the starting materials that are selected and reaction conditions. In embodiments, covalent bonding of the PGL to the substrate surface or modified substrate surface, and covalent bonding of the immobilized protein on the PGL modified substrate surface is preferred for stability and reproducibility considerations.

A "poor" solvent refers to a solvent that is a weak or a non-solvent for PGL and provides superior immobilization surfaces compared to PGL modified substrate surfaces prepared with a "good" solvent. A "good" solvent refers to a solvent that is a strong solvent for PGL and results in thin PGL films rather than the desired PGL nanoparticulate decorated surfaces having superior immobilization properties and capacity.

The indefinite article "a" or "an" and its corresponding definite article "the" as used herein means at least one, or one or more, unless specified otherwise.

"Include," "includes," or like terms means including but not limited to.

"About" modifying, for example, the quantity of an ingredient in a composition, concentrations, volumes, process temperature, process time, yields, flow rates, pressures, and like values, and ranges thereof, employed in describing the embodiments of the disclosure, refers to variation in the numerical quantity that can occur, for example, through typical measuring and handling procedures used for making compounds, compositions, concentrates or use formulations; through inadvertent error in these procedures; through differences in the manufacture, source, or purity of starting materials or ingredients used to carry out the methods; and like considerations. The term "about" also encompasses amounts that differ due to for example aging of a formulation with a particular initial concentration or mixture, and amounts that differ due to mixing or processing a formulation with a particular initial concentration or mixture. Whether modified by the term "about" the claims appended hereto include equivalents to these quantities.

"Consisting essentially of" in embodiments refers, for example, to a surface composition, a method of making or using a surface composition, formulation, or composition on the surface of a substrate or surface, such as a microplate or a biosensor, and articles, devices, or apparatus of the disclosure, and can include the components or steps listed in the claim, plus other components or steps that do not materially affect the basic and novel properties of the compositions, articles, apparatus, and methods of making and use of the disclosure, such as particular reactants, particular additives or ingredients, a particular agent, a particular surface modifier or condition, a particular ligand candidate, or like structure, material, or process variable selected. Items that may materially affect the basic properties of the components or steps of the disclosure or may impart undesirable characteristics to aspects of the present disclosure include, for example, decreased affinity of protein or like analyte molecules for the modified surface, decreased reactivity of the aldehyde modified surface for analyte molecules, and like characteristics.

"Optional" or "optionally" or like terms generally refer to, for example, that the subsequently described event or circumstance can or cannot occur, and that the description includes instances where the event or circumstance occurs and instances where it does not.

"Contact" or "contacting" or like terms refer to, for example, an instance of exposure by close physical contact of at least one substance to another substance.

Thus, the claimed invention may suitably comprise, consist of, or consist essentially of: a nanoparticulate composition including a polymer having aldehyde presenting surface groups; a method of making an article having a PGL nanoparticulate modified surface; a method of immobilizing a biomolecule including contacting an article having PGL nanoparticulate modified surface as defined herein with a biomolecule; an article having a PGL nanoparticulate modified surface; and an apparatus for LID including an optical biosensor having a PGL nanoparticulate modified contact surface.

In embodiments the disclosure provides a polyglutaraldehyde modified substrate and methods of making and using the substrates for binding or immobilizing biomolecules. In embodiments the disclosure also provides an article and apparatus for immobilizing biomolecules and methods therewith for label independent detection of the immobilization. The article can include a substrate having polyglutaraldehyde (PGL) nanoparticles associated with the substrate's surface.

In embodiments the disclosure provides a method of making an article, the method comprising:
providing a functionalized substrate;
contacting the functionalized substrate with a polyglutaraldehyde nanoparticulate formulation to deposit polyglutaraldehyde nanoparticles on the functionalized substrate.

The functionalized substrate can comprise, for example, a glass sheet or like form having a surface treated with a surface substantive material such as an amino-hydrocarbylsilane, a hydrazido-hydrocarbylsilane, an amino-hydrocarbyl silsesquioxane, a hydrazido-hydrocarbyl sisesquioxane and like compounds, or combinations thereof. Such a material generally responds to formula (I) below:

(X)₃Si-R¹-NR²R³ (I)

where
X is a leaving group independently selected from halogen (-Cl, -Br, -I, -F), or -OR where R is independently hydrogen, or a monovalent hydrocarbyl group;
R¹ is a divalent hydrocarbyl group;
R² is independently hydrogen, or a monovalent hydrocarbyl group;
R³ is independently hydrogen, a monovalent hydrocarbyl group, or -NR²R⁴, where R⁴ is independently hydrogen, a monovalent hydrocarbyl group; and salts thereof.

Compounds of formula (I) or suitable alternative or equivalent compounds are commercially available or can be readily prepared, see for example, Pludemann, Silane Coupling Agents, (1982), and Gelest, Inc., (www.gelest.com).

"Hydrocarbon," "hydrocarbyl" and like terms, in the context of the tie-layer compounds of the disclosure, refer to divalent -R¹- moieties, and can include, for example, alkyl hydrocarbons, aromatic or aryl hydrocarbons, alkyl substituted aryl hydrocarbons, alkoxy substituted aryl hydrocarbons, heteroalkyl hydrocarbons, heteroaromatic or heteroaryl hydrocarbons, alkyl substituted heteroaryl hydrocarbons, alkoxy substituted heteroaryl hydrocarbons, and like hydrocarbon moieties, and as illustrated herein. In embodiments, the hydrocarbon of the amino-hydrocarbylsilane compound, the hydrazido-hydrocarbylsilane compound, and like compounds, can be selected to be the same, similar to, or at least chemically or physically compatible with hydrocarbons, if any, contained in or on the substrate, such as an organic polymer, an inorganic polymer such as a glass, an organic-inorganic hybrid polymer such as a organo substituted polysiloxane, or combinations thereof. R¹ is a divalent spacer group selected from certain hydrocarbyl groups, such as aliphatic and aromatic moieties, for example, saturated or unsaturated -(C₁-C₂₀) alkylene-, (C₁-C₄)alkyl substituted saturated or unsaturated -(C₁-C₂₀) alkylene-, -Ar-, (C₁-C₁₀)alkyl substituted -Ar-, -(CH₂)ₖ-Ar-(CH₂)ₖ-, (C₁-C₄)alkyl substituted -(CH₂)ₖ-Ar-(CH₂)ₖ-, - Ar-Ar-, (C₁-C₄)alkyl substituted -Ar-Ar-, -(CH₂)ₖ-Ar-Ar-(CH₂)ₖ-, (C₁-C₄)alkyl substituted -(CH₂)ₖ-Ar-Ar-(CH₂)ₖ-, -(CH₂)ₖ-Ar-(CH₂)ₖ-Ar-(CH₂)ₖ-, (C₁-C₄)alkyl substituted -(CH₂)ₖ-Ar-(CH₂)ₖ-Ar-(CH₂)ₖ-, -Ar-O-Ar-, (C₁-C₄)alkyl substituted -Ar-O-Ar-, -(CH₂)ₖ-Ar-O-Ar-(CH₂)ₖ-, (C₁-C₄)alkyl substituted -(CH₂)ₖ-Ar-O-Ar-(CH₂)ₖ-, Het, (C₁-C₄)alkyl substituted -Het-, -(CH₂)ₖ-Het-, (C₁-C₄)alkyl substituted -(CH₂)ₖ-Het-, -(CH₂)ₖ-Het-(CH₂)ₖ-, (C₁-C₄)alkyl substituted -(CH₂)ₖ-Het-(CH₂)ₖ-, -Ar-(CH₂)ₖ-, (C₁-C₄)alkyl substituted -Ar-(CH₂)ₖ-, Ar'-CH=, or (C₁-C₄)alkyl substituted Ar'-CH=, where k is an integer from 1 to about 20.

Specific and preferred values listed below for radicals, substituents, and ranges, are for illustration only; they do not exclude other defined values or other values within defined ranges for the radicals and substituents. The compounds of the disclosure include compounds of formula (I) and like compounds having any combination of the values, specific values, more specific values, and preferred values described herein.

Specifically, C₁₋₄alkyl can be methyl, ethyl, propyl, isopropyl, butyl, iso-butyl, sec-butyl, tert-butyl; C₁₋₇alkyl can be methyl, ethyl, propyl, isopropyl, butyl, iso-butyl, sec-butyl, tert-butyl, pentyl, 3-pentyl, hexyl, or heptyl; (C₃₋₁₂)cycloalkyl can be cyclopropyl, cyclobutyl, cyclopentyl, cyclohexyl, cycloheptyl, cyclooctyl, bicyclic, or multi-cyclic substituents, such as of the formulas 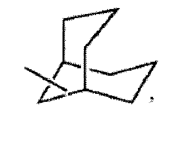 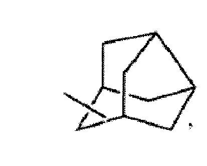 and

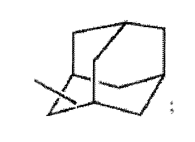

C₁₋₇alkoxy can be methoxy, ethoxy, propoxy, isopropoxy, butoxy, iso-butoxy, secbutoxy, pentoxy, 3-pentoxy, hexyloxy, 1-methylhexyloxy, or heptyloxy; -C(=O)alkyl or (C₂₋₇)alkanoyl can be acetyl, propanoyl, butanoyl, pentanoyl, 4-methylpentanoyl, hexanoyl, or heptanoyl; aryl (Ar) can be phenyl, naphthyl, anthracenyl, phenanthrenyl, fluorenyl, tetrahydronaphthyl, or indanyl; Het can be pyrrolidinyl, piperidinyl, morpholinyl, thiomorpholinyl, or heteroaryl; and heteroaryl can be furyl, imidazolyl, triazolyl, triazinyl, oxazoyl, isoxazoyl, thiazolyl, isothiazoyl, pyrazolyl, pyrrolyl, pyrazinyl, tetrazolyl, pyridyl, (or its N-oxide), thienyl, pyrimidinyl (or its N-oxide), indolyl, isoquinolyl (or its N-oxide), or quinolyl (or its N-oxide).

Specifically, -(CH₂)ₖ- can be a -(C₁₋₂₀alkylene)- when k is an integer from 1 to about 20, which can be methylenyl, ethylenyl, propylenyl, butylenyl, pentylenyl, 3-pentylenyl, hexylenyl, heptylenyl, octylenyl, nonylenyl, decylenyl, and like homologs.

Specifically, -(CH₂)ₖ- can be a -(C₁₋₄alkylene)- when k is an integer from 1 to about 4, which can be methylenyl, ethylenyl, propylenyl, or butylenyl.

A specific value for Het includes a five-(5), six-(6), or seven-(7) membered saturated or unsaturated ring containing 1, 2, 3, or 4 heteroatoms, for example, non-peroxide oxy, thio, sulfinyl, sulfonyl, and nitrogen; as well as a radical of an orthofused bicyclic heterocycle of about eight to twelve ring atoms derived therefrom, particularly a benz-derivative or one derived by fusing a propylene, trimethylene, tetramethylene, or another monocyclic Het diradical thereto.

A specific compound is of the formula (I) wherein R¹ can be, for example, - Ar-, or (C₁-C₄)alkyl substituted -Ar-,where Ar can be, for example, an ortho-, meta-, or para-substituted -C₆H₄-;

Another specific compound is of the formula (I) wherein R¹ can be, for example, -CH₂-Ar-CH₂-, or (C₁-C₄)alkyl substituted -CH₂-Ar-CH₂-,where Ar can be, for example, an ortho-, meta-, or para-substituted -C₆H₄-;

Another specific compound is of the formula (I) wherein R¹ can be, for example, -Ar-CH₂-, or (C₁-C₄)alkyl substituted -Ar-CH₂-, where Ar can be, for example, an ortho-, meta-, or para-substituted -C₆H₄-; such as -C₆H₄-CH₂-, or (C₁-C₄)alkyl substituted -Ar-CH₂-, such as -C₆H₃(R)-CH₂-, or optionally alkyl substituted -C₆H₄-CH(R)-, where R can be (C₁-C₄)alkyl or a substituted (C₁-C₄)alkyl.

Another specific compound is of the formula (I) wherein R¹ can be, for example, Ar'-CH=, or (C₁-C₄)alkyl substituted Ar'-CH=, where each Ar' is an aryl substituent connected to the main hydrocarbyl chain, such as C₆H₄-CH=, C₆H₃(R)-CH=, or C₆H₄-C(R)=, where R can be (C₁-C₄)alkyl or substituted (C₁-C₄)alkyl.

Another specific compound is of the formula (I) wherein R¹ can be saturated or unsaturated -(C₂-C₆)alkylene-, -Ar-, or (C₁-C₄)alkyl substituted -Ar-.

Another specific compound is of the formula (I) wherein R¹ can be saturated or unsaturated -(C₂-C₆) alkylene-, -C₆H₅-, or (C₁-C₄)alkyl substituted -C₆H₅-.

Another specific compound is of the formula (I) wherein R¹ can be propyl -CH₂-CH₂-CH₂- or -C₆H₅-; X can be chloro; and R² and R³ are independently -H or (C₁-C₄)alkyl.

Another specific compound is of the formula (I) wherein R¹ can be undecyl -(CH₂-)₁₁-; R² can be -H, -Me, Et, Pr, Bu, i-Bu, s-Bu, or t-Bu; X can be chloro or bromo; and R³ is independently -H, a monovalent hydrocarbyl group such as (C₁-C₄)alkyl, or -NR²R⁴, where R² is as defined above and R⁴ is independently hydrogen, a monovalent hydrocarbyl group; and salts thereof.

A specific compound of the formula (I) is an amino-hydrocarbylsilane of the formula:

(CH₃O)₃Si-(CH₂)₃-NH₂

Another specific compound of the formula (I) is of the formula:

(CH₃O)₃Si-(CH₂)₁₁-NH-NH₂

Another specific compound of the formula (I) is of the formula:

(CH₃O)₃ Si-(CH₂)₃-C₆H₅-(CH₂)₃-NH₂

Another specific compound of the formula (I) is of the formula:

(CH₃O)₃Si-CH₂-C₆H₅-SO₂-NH-NH₂

Hydrazides are compounds having a functional group characterized by a nitrogen to nitrogen covalent bond with 4 substituents with at least one of them is an acyl group or like group, such as -C(=O)-, or a -S(=O)-, -S(=O)₂-, for example, sulfonylhydrazides, such as p-toluenesulfonylhydrazide. The general structure for an hydrazide is, for example, R₁R₂N-NR₃R₄. A related class of compounds are called hydrazines and do not carry an acyl group.

In embodiments the disclosure provides a method of making an article, the method comprising: providing an aminated substrate; contacting the aminated substrate with a PGL formulation to deposit PGL nanoparticles on the aminated substrate. The aminated substrate can be, for example, a glass slide or glass plate or like substrate or support having a surface treated with aminopropylsilane, or like amino functional silane.

In embodiments the polyglutaraldehyde nanoparticulate formulation comprises polyglutaraldehyde nanoparticles in a mixture of H₂O:DMSO in a volume ratio of from about 100:0 to about 80:20. The polyglutaraldehyde nanoparticulate formulation can comprise from about 1 to about 5 wt% polyglutaraldehyde nanoparticles suspended in H₂O. The polyglutaraldehyde nanoparticulate formulation can also comprise polyglutaraldehyde nanoparticles, a liquid comprised of H₂O, or a mixture of H₂O and DMSO, and a water soluble base.

In embodiments the PGL formulation can be, for example, from about 1 to about 5 wt % PGL and a mixture of solvents that can be, for example, DMSO and H₂O having a high water to DMSO ratio. In embodiments the solvent mixture can be, for example, from about 2 to about 4 wt% PGL nanoparticles and the solvent mixture comprises H₂O. In embodiments the PGL formulation can include a reducing agent in an amount of from about 0.01 wt% to about 1 wt%, such as NaCNBH₃ or like reducing agents, a surfactant, such as SDS or like surface active agents, and combinations thereof. In embodiments the substrate, alternatively or additionally, can have hydrazide groups on the substrate surface.

In embodiments, there is provided a nanoparticulate comprising: a polymer having aldehyde presenting surface groups. In embodiments, the polymer can comprise a polyglutaraldehyde having at least one of a saturated aldehyde, an unsaturated aldehyde, or mixtures thereof.

In embodiments the disclosure provides an article, more particularly suitable for label-independent detection, the article comprising a substrate comprising a contact surface; the contact surface comprising polyglutaraldehyde nanoparticles deposited directly on the substrate or comprising a tie layer having polyglutaraldehyde nanoparticles deposited on the tie layer.

In embodiments the disclosure provides an article, more particularly suitable for label-independent detection, the article comprising: a substrate comprising a contact surface comprising a tie layer, such as an aminated layer or like surface layer, and having polyglutaraldehyde nanoparticles deposited on the tie layer. The substrate can be, for example, at least one of a glass, a polymer, a composite, or combinations thereof and the PGL nanoparticulates can have a surface coverage on the article of from about 0.01 % to about 10% and can have a particle diameter of from about 1 to about 1,000 nanometers, and from about 50 to about 150 nanometers, including intermediate or overlapping particle diameter ranges. The polyglutaraldehyde of the nanoparticles has at least one of a saturated aldehyde, an unsaturated aldehyde, or mixtures thereof.

In embodiments the disclosure provides an article, more particularly suitable for label-independent detection, the article comprising:
a substrate comprising a contact surface comprising polyglutaraldehyde nanoparticles, the nanoparticles can be, for example, covalently or ionically associated with the substrate. The particles can also be deposited on the substrate. The substrate can be, for example, an aminated material or an amine presenting surface, such as at least one of: an aminated plastic, an aminated glass, an aminated composite, or like forms, and combinations thereof. An aminated material or an amine presenting surface can be obtained by, for example, contacting the substrate, such as a glass, with ammonia. The aminated surface or aminated material can they be decorated with the polyglutaraldehyde nanoparticles using, for example, the solvent mixture as described above and as demonstrated herein.

In embodiments the disclosure provides a method of immobilizing a biomolecule, the method comprising: contacting the aforementioned article with a biomolecule; and optionally rinsing and drying the contacted article. The biomolecule can be, for example, at least a protein, a nucleic acid, a pathogen, a cell structural component, a cell, or combinations (mixtures thereof), more particularly mixtures of proteins.

In embodiments the disclosure provides an apparatus for label independent detection, the apparatus comprising: an optical biosensor having the aforementioned article having PGL nanoparticles deposited on the contact surface.

### Supports and Methods of Making Thereof

Described herein are supports useful for performing assays. In embodiments a support for performing an assay comprises a substrate and a PGL polymer directly or indirectly attached to the substrate, where the PGL polymer has a plurality of reactive groups capable of attaching to a biomolecule, and the PGL polymer does not contain a photoreactive group.

In embodiments the disclosure provides a method for making a support for performing an assay, the method comprising: attaching a PGL polymer, such as PGL nanoparticulates, directly or indirectly to a substrate, the PGL polymer has a plurality of reactive groups capable of attaching to a biomolecule, and the PGL polymer does not contain a photoreactive group.

### a. Substrate

The substrate can be any suitable substrate and can include, for example, a microplate, a slide, or any other material that is capable of attaching to the binding polymer. In embodiments when the substrate is a microplate, the number of wells and well volume can vary and depend upon the scale and scope of the analysis. Other examples of useful substrates include, for example, a cell culture surface such as a 384-well microplate, a 96-well microplate, 24-well dish, 8-well dish, 10 cm dish, a T75 flask, and like substrate articles.

For optical or electrical detection applications, the substrate can be transparent, impermeable, or reflecting, as well as electrically conducting, semiconducting, or insulating. For biological applications, the substrate material can be either porous or nonporous and can be selected from either organic or inorganic materials.

In embodiments, the substrate can be, for example, a plastic, a polymeric or co-polymeric substance, a ceramic, a glass, a metal, a crystalline material, a noble or semi-noble metal, a metallic or non-metallic oxide, an inorganic oxide, an inorganic nitride, a transition metal, and like materials, or any combination thereof.

Additionally, the substrate can be configured so that it can be placed in any detection device. In embodiments, sensors can be integrated into the bottom or underside of the substrate and used for subsequent detection. These sensors could include, for example, optical gratings, prisms, electrodes, quartz crystal microbalances, and like devices. Detection methods can include, for example, fluorescence, phosphorescence, chemiluminescence, refractive index, mass, electrochemical, and like methods, or a combination thereof. In embodiments the substrate can be a resonant waveguide grating sensor.

In embodiments, the substrate can be comprised of an inorganic material. Examples of inorganic substrate materials can include metals, semiconductor materials, glass, and ceramic materials. Examples of metals that can be used as substrate materials include gold, platinum, nickel, palladium, aluminum, chromium, steel, and gallium arsenide. Semiconductor materials useful for the substrate material can include, for example, silicon and germanium. Glass and ceramic materials useful for the substrate material can include, for example, quartz, glass, porcelain, alkaline earth aluminoborosilicate glass, and other oxides or mixed oxides. Further examples of inorganic substrate materials include graphite, zinc selenide, mica, silica, lithium niobate, and inorganic single crystal materials. In embodiments, the substrate can be made of gold such as, for example, a gold sensor chip.

In embodiments, the substrate can comprise a porous, inorganic layer. Any of the porous substrates and methods of making such substrates disclosed in U.S. Patent No. 6,750,023, can be used for example. In embodiments, the inorganic layer on the substrate comprises a glass or metal oxide. In embodiments, the inorganic layer can comprise a silicate, an aluminosilicate, a boroaluminosilicate, a borosilicate glass, and like silicates, or a combination thereof. In embodiments, the inorganic layer can comprise, for example, TiO₂, SiO₂, Al₂O₃, Cr₂O₃, CuO, ZnO, Ta₂O₅, Nb₂O₅, ZnO₂, and like oxides, or a combination thereof. In embodiments, the substrate can comprise SiO₂ with a layer comprising Ta₂O₅, Nb₂O₅, TiO₂, Al₂O₃, silicon nitride, or a mixture thereof, where the layer is adjacent to the surface of the SiO₂. The silicon nitride can be represented by the formula SiNₓ, where the stoichiometry of silicon and nitrogen can vary.

In a further aspect, the substrate can be composed of an organic material. Useful organic materials can be polymeric materials due to their dimensional stability and resistance to solvents. Examples of organic substrate materials include, for example, polyesters, such as polyethylene terephthalate, polybutylene terephthalate, polyvinylchloride, polyvinylidene fluoride, polytetrafluoroethylene, polycarbonate, polyamide, poly(meth)acrylate, polystyrene, polyethylene, ethylene/vinyl acetate copolymer, and like polymers, or combinations thereof.

In embodiments the disclosure provides an article for label independent detection, the article comprising: a substrate comprising a contact surface comprising polyglutaraldehyde nanoparticles, the nanoparticles being covalently bonded to the substrate. The substrate can comprise, for example, at least one of: an aminated plastic, an aminated glass, an aminated composite, and like aminated surfaces, or combinations thereof. In embodiments, the substrate can be comprised of a material that possesses functional groups capable of attaching one or more biomolecules. For example, the substrate can be comprised of one or more binding polymers described herein and molded into any desired shape. In embodiments, the biomolecule and other components can be attached directly or indirectly to the substrate. In embodiments, the substrate can possess or be processed to possess functional groups, such as amine groups obtained, for example, by an amination process or other chemical modification to avoid an intermediate tie-layer.

### b. Binding Polymer

In various aspects, a PGL binding polymer nanoparticulate comprises one or more reactive groups that can bind a biomolecule directly or indirectly to the substrate. The reactive groups of the PGL binding polymer permit the attachment of the biomolecule to the PGL polymer. In embodiments, the PGL binding polymer can be attached to the substrate covalently, electrostatically, or both. The PGL binding polymer can have one or more different types of reactive groups. It is also contemplated that two or more different type of PGL binding polymers can be attached to the substrate.

In embodiments, the reactive group is capable of forming a covalent bond with a nucleophile such as, for example, an amine or thiol. The amine or thiol can be, for example, part of the biomolecule or a molecule that is attached to the surface of the substrate (i.e., a tie-layer) and used to indirectly attach the PGL binding polymer to the substrate.

The PGL binding polymer can be either linear or non-linear. A non-linear PGL binding polymer can be, for example, branched, hyperbranched, crosslinked, a dendritic polymer, and like polymers, or a combination thereof. The PGL binding polymer can be a homopolymer or a copolymer.

In embodiments the disclosure provides methods for preparing surfaces capable of immobilizing receptors (e.g., proteins) at high density. The LID biosensor in accordance with the disclosure has a high sensitivity for the detection of bio-molecular recognition events. In addition, the substrate and immobilization methodologies can be accomplished without any pre-activation. Pre-activation methods are generally recognized as time consuming and a source of variability.

Although the composition and method of the disclosure may be useful for detection using labeled ligands it is particularly well suited for biosensors based on label-free or label independent detection (LID) methods such as a resonant waveguide (RWG) optical biosensor, for example, Coming Incorporated's Epic^{®} system or those based on surface plasmon resonance (SPR).

For such techniques based on the local change of refractive index induced by the adsorption of a ligand onto an immobilized receptor, a surface chemistry that enhances a biosensor's signal by increasing the number of the immobilized receptors and can thus increase the capture of a greater number of ligands would be useful. If the receptor is, for example, a protein, the biosensor surfaces prepared in accordance with the disclosure can detect bio-molecular recognition events even when the protein is immobilized at concentrations as low as, for example, about 1.5 microgram/mL.

In embodiments of the disclosure, the surface chemistry of the sensor for label-free or label-independent detection can include, for example, depositing a complete or partial coating of PGL nanoparticulates (colloidal particles) bearing reactive groups, such as an aldehyde, and like groups, or combinations thereof. Preferably, the reactive groups are polymeric or oligomeric aldehydes, and more preferably the aldehyde can be or contain a polymeric or oligomeric unsaturated aldehyde, an α,β-unsaturated aldehyde, an α-hydroxy substituted aldehyde, or a mixture thereof.

Immobilization chemistry based on anhydride groups is particularly useful. It is known that receptors, such as proteins, have functional groups that are able to react with an anhydride including the alpha-amine at the N-terminals, the epsilon-amine of lysine side chains, cysteine sulfhydryl groups, phenolate ion of tyrosine residues, and the imidazolyl ring of histidines (ref. 1). Therefore a PGL copolymer containing anhydride moieties can be particularly suitable to immobilize such biomolecules. In addition, the high reactivity of the anhydride moieties at neutral pH allows an attachment of an anhydride copolymer onto the sensor surface under mild conditions, providing that an adhesion layer was previously applied to the sensor substrate.

Although the modification of surfaces with maleic anhydride copolymers for the immobilization of biomolecules, such as DNA, proteins, peptides, and sugars, has been reported (refs. 2, 3, and 4), commonly owned and assigned US Patent Application Publication 2006/0110594, USSN 10/996,952, filed Nov. 24, 2004, (ref. 5) disclosed LID sensors having pre-activated surface chemistry based on a maleic anhydride copolymer bound to the sensor surface. Unfortunately, despite the high reactivity of the anhydride groups, the amount of the receptors (e.g., proteins) bound to the sensor surface was low. Therefore the sensitivity of the sensors was lower than expected and issues remain so as to be compatible with high throughput screening experiments such those performed for drug discovery requiring high sensitivity detection. Moreover a major drawback of anhydride based coupling chemistries is the high sensitivity to hydrolysis which leads to difficulties in preparing surfaces having reproducible binding capacity. Each anhydride group may breakdown by reacting with one molecule of water to yield to two carboxylic groups that may be unable to bind covalently the proteins. This uncontrolled loss of reactivity for covalent coupling is a significant drawback. Moreover, these generated carboxylic groups may negatively interact with proteins by means of non-specific interactions. These non-specific interactions may add variability to the immobilization capacity and potential non-covalent immobilization of the receptor. Because of the potential that a nonspecifically adsorbed receptor may induce a shift of the detection signal, this non-covalent adsorption can be a significant obstacle for a LID sensor. To overcome these drawbacks U.S. Provisional Patent Application 60/754,747 (ref. 6) has disclosed surface chemistry comprising a modified maleic anhydride copolymer bound to the surface of the LID biosensor. The modified maleic anhydride copolymer is obtained by reaction between the maleic anhydride copolymer and a reactant capable of reacting with the anhydride group leading to the opening of the anhydride group. In embodiments, the compound may contain at least one group selected from, for example, an amine, an alcohol, a thiol, and like groups, or combinations thereof. Although, this chemistry is particularly useful to immobilize large amount of proteins, some important issues are encountered.

Firstly, due to the presence of unwanted negative electric charges resulting of the hydrolysis process of the anhydride groups, protein are immobilized in a greater yield when they are dissolved in a buffer having a pH below the isoelectric point (pI) of the protein whereas poor immobilization capacity is observed when the immobilization is performed at a pH far above this pI. This phenomenon makes the immobilization process pH and pI dependent which can be a significant limitation. In addition, the immobilized proteins can strongly interact with the sensor surface due to these negative charges and though the immobilization capacity is enhanced, the immobilized protein may suffer from strong interactions with the sensor surface and may be denatured. Thus, the strong interaction between the protein and the surface may change the conformation of the protein making the binding event of small molecules on these immobilized proteins more difficult or impossible, if not distorted from native binding.

Secondly, the proteins to be immobilized must be used at rather high concentrations to promote the immobilization process which competes with hydrolysis. This high protein concentration requirement may be an issue when the proteins are scarce, precious, or both.

As an alternative technique, a reductive amination reaction using an aldehyde as a reactive group has been described as a way to immobilize protein independent of their pI and to some extent the pH at which the immobilization is performed. In that situation protein immobilization occurs due to the reaction between amino groups of the protein and the aldehyde group (ref. 7). Unfortunately, this approach also has significant drawbacks. The reaction of an aldehyde with the amino group of the biomolecule forms a reversible Schiff base. This interaction can be enhanced at slightly alkaline pH but the Schiff base is readily broken down by equilibrium dynamics. Therefore an additional reduction step is needed to convert the labile Schiff base to a stable secondary or tertiary amine linkage for an efficient and stable protein immobilization. This reduction step is generally performed by adding a reducing agent such as sodium borohydride (NaBH₄) or the milder sodium cyanoborohydride (NaCNBH₃) in the course of the immobilization step or just after the immobilization. In either instance, this additional reduction step is time consuming and the chemicals involved are potentially hazardous and require appropriate handling.

A technique to obviate the additional reduction step is known and uses a conjugated unsaturated aldehyde instead of a saturated aldehyde. Such unsaturated aldehydes are, for example, found in the structure of the polymerized form of the glutaraldehyde, i.e., polyglutaraldehyde (ref. 8). Evidence suggests that the conjugated aldehyde moieties in the polymer give rise to stable reactions products whereas monomeric (saturated) glutaraldehyde yields hydrolysable labile entities (ref. 9). Synthesis of polyglutaraldehyde is known, see for example, U.S. Patent No. 6,326,136 B1 (ref. 10) and U.S. Patent No. 4,369,226 (ref. 11). Unfortunately, even if the stability of the reaction product is improved using an unsaturated aldehyde and avoids a reducing agent, protein immobilization capacity can remain poor and is not a good candidate as surface chemistry for label-free detection techniques.

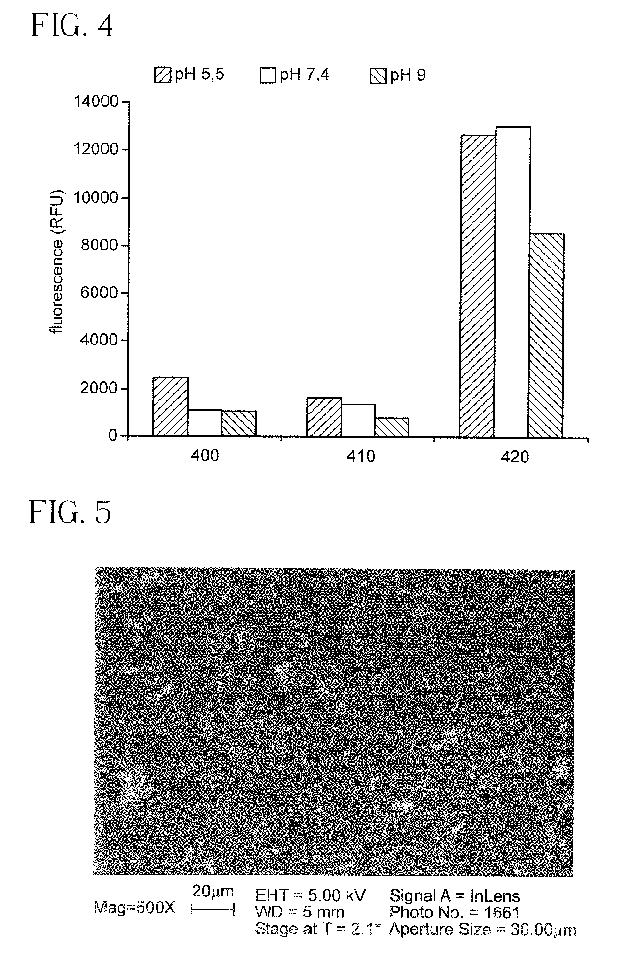

Aldehyde surface chemistry can be obtained using numerous known derivatization techniques. For example, epoxy silane coated surfaces can be easily converted to aldehyde surfaces by firstly hydrolyzing the epoxy groups to the corresponding cis-diol and then oxidizing the cis-diol to the aldehyde. However, this approach produces very thin monolayer-like surfaces which may have poor protein capture efficiency (see comparative examples and Fig. 4). To improve protein capture, a polymer bearing aldehyde groups can be preferred because of their higher aldehyde content. Aldehyde polymers can be prepared, for example, by mild oxidation of a polysaccharide, such as dextran, using sodium metaperiodate as oxidizing agent. Other synthetic polymers bearing aldehyde groups can be obtained from, for example, free radical polymerization of (meth)acrolein monomers or aldol condensation under alkaline conditions of a dialdehyde such as glutaraldehyde (H-C(=O)-(CH₂)₃-C(=O)-H).

Controlled oxidation of dextran is the most common technique used to prepare polyaldehyde. Unfortunately, even if the preparation of the polymer is simple, its use as a surface for label-free detection is still problematic due to its very low protein-immobilization capacity. The low immobilization efficiency is generally attributed to the low grafting efficiency of the oxidized dextran onto the sensor surface. This low grafting yield is the most common issue associated with the "grafting to" technique (ref. 12). In addition, the aldehyde content of the oxidized polyaldehyde dextran is also limited.

To circumvent the issue encountered with oxidized dextran, Ho, et al., in U.S. Patent No. 6,733,894 B2 (ref. 13) mentions use a grafted polymer, such as PVA-g(Allyl Alcohol-co-Acrolein) to prepare high capacity aldehyde microarrays for biomolecules. Unfortunately, the polymer synthesis requires the use of (meth)acrolein which is difficult to polymerize and may include unreacted monomer. The presence of unreacted monomers generally includes an odor and toxicity that may require extensive purification or precautions. U.S. Patent No. 5,543,456 (ref. 14) mentions the residual monomer problem when (meth)acrolein was used to prepare aqueous resin dispersion. The unreacted (meth)acrolein must be removed from the reaction media with an additional step that is time consuming and impacts the cost of the final product.

Additionally, when a homopolymer of acrolein is employed, it is generally difficult to dissolve because of the highly branched structure of the polymer. Moreover a strong solvent such as pyridine or DMF is generally required to dissolve the polyacrolein and may lead to degradation of solvent sensitive substrates or supports.

Despite the advantages provided by the use of aldehyde surface chemistry for biomolecule capture, the level of immobilization performance obtained with commercially available aldehyde-coated surfaces or those made according to published descriptions are incompatible with LID applications (see, e.g., Fig. 4). Although not limited by theory, the poor immobilization performances of available polyaldehyde coatings are likely due to the aldehyde being a monolayer or very thin layer of aldehyde material which provides a limited specific area and thus limited immobilization capacity.

Consequently, the present disclosure provides biomolecule immobilization surfaces having pre-activated chemistry and having a very high immobilization capacity which is compatible with label-free detection sensors. The immobilization surfaces provide a charge neutral surface to avoid non-specific adsorption of protein, and hydrolysis resistance which is high enough to provide long shelf-life and good immobilization coupling yields even at low protein concentration, and especially where the protein is precious.

In embodiments the disclosure provides a label-free detection optical biosensor having a surface comprising a partial coating of polyaldehyde nanoparticulates, for example, polyglutaraldehyde nanoparticles deposited from a liquid carrier vehicle or solvent mixture, to create a surface condition or roughness on the sensor surface which enhances the biomolecule immobilization capacity of the surface.

Deposition experiments of polyglutaraldehyde from pure DMSO and DMSO/water mixtures containing a high DMSO content, such as 50% solvent vol. or more yielded a surface having a protein immobilization capacity which was low and not compatible with label free detection techniques despite the intrinsic properties of polyglutaraldehyde (i.e., very high aldehyde content and an unsaturated hydrocarbon backbone that does not require a reduction step for stable protein coupling). Thus, strong PGL solvents or solvent mixtures yielded coatings which were smooth and uniform but which did not out-perform immobilization capacity of, for example, available aldehyde silane coatings or oxidized dextran coatings, and were thus not compatible with label-free detection. However, when an appropriate weak mixed solvent composition or neat weak solvent of the disclosure was used for coatings, it was discovered that an unexpectedly large amount, such as from about 6 to about 10 fold increase, of protein could be subsequently immobilized on the sensor surface.

It is known that monomeric glutaraldehyde and low molecular weight polyglutaraldehyde (PGL) polymers are water soluble whereas when the PGL polymer reaches an appropriate molecular weight, it becomes water insoluble. For example, polyglutaraldehyde having a molecular weight of 20,000 is insoluble in pure water (poor or weak solvent) but is fully soluble in DMSO (good or strong solvent). Thus, in embodiments a poor solvent is water and a good solvent is DMSO. Both H₂O and DMSO are compatible with LID biosensors materials.

U.S. Patent No. 4,267,234, mentions a method to prepare polyglutaraldehyde (PGL) and more particularly microparticles or microspheres (20 nanometers to 10 micrometers) of polyglutaraldehyde and use of the particles as protein binding substrates. Proteins such as antibodies, immunoglobulins, and like proteins, can be linked to the prepared microspheres and used as a suspension.

Unfortunately, binding events on protein immobilized on a polyaldehyde-carrier in suspension can not be use on a surface oriented LID platform such as Epic^{®} or SPR. The SPR or Epic^{®} system detects refractive index changes close to the biosensor surface. Generally the accumulation of 1 picogram of protein per mm² corresponds roughly to 1 RU response or 1 pm shift on SPR and Epic^{®} respectively. The protein on which a small molecule may bind should be confined close to the surface of the sensor and should remain within the maximum distance probed by the evanescent field wave. Typically this distance is, for example, less than about 200 nm. This distance requirement for effective sensing limits the use of particles having diameter above about 200 nm. Useful particles are thus nanoparticles having a diameter lower than about 200 nm and more preferably about 100 nm.

In embodiments, a weak solvent or weak solvent mixture, that is a balanced combination of poor and good solvents (poor/good solvent mixture), and optional use of a surfactant in combination with the solvent mixture permits the preparation and biosensor surface deposition of PGL nano-particles having the appropriate particle size range. A useful solvent mixture can be, for example, H₂O:DMSO of from about 100:0 to about 80:20 by weight or by volume.

A useful surfactant can be, for example, sodium dodecyl sulfate, and like surfactants, tensides, and detergents, or combinations thereof. A useful surfactant amount in combination with the solvent or solvent mixture can be, for example, from about 0.001wt% to about 1 wt% based on the total weight of the solvent or solvent mixture and the optional surfactant.

In embodiments, the LID biosensor of the disclosure can comprises a substrate coated with a first tie-layer, such as a layer bearing amino or hydrazine groups, on which nanoparticles made of polyaldehyde are deposited and decorate only a portion of the sensing surface. The sensor may be, for example, an Epic^{®} plate coated with an aminopropylsilsesquioxane or like amino silane layer and decorated with polyglutaraldehyde nanoparticles that are firmly attached on the tie-layer. Here anchoring can occur through a covalent bond between an aldehyde group of the polyaldehyde and an amino group of the tie-layer. Because the polyglutaraldehyde of the disclosure is composed of mainly unsaturated aldehyde groups no reduction step is typically required for immobilization. However, to insure a stable linkage between the particles and the sensor surface, a reduction agent and reduction step can optionally be included following the polyglutaraldehyde particle surface deposition-decoration step.

In embodiments, the disclosure provides a method for making optical biosensors having very high protein immobilization capacity, such as from about 50 to about 100% capacity, that can provide a high sensitivity to biomolecular recognition events that use label free detection methods. The disclosure provides methods for making optical sensors for label free detection using very low protein concentrations which means that the cost of analysis can be drastically reduced compared to other LID techniques. Typically biomolecular recognition events can be monitored using protein immobilization at, for example, about 1.5 microgram/mL which means that the protein consumption can be, for example, about 30 nanograms/well in a 384-well plate.

The biosensor made according to the disclosure can be pre-activated and does not require an activation procedure. The high immobilization capacity surface of the article of the disclosure does not require any additional stabilization or reduction step as is generally done for protein immobilization using available aldehyde based chemistries.

The disclosure provides a neutral surface which has low sensitivity to hydrolysis. This surface is readily adapted to various substrates and is compatible with any label-free detection platform, such as based on SPR or resonant gratings, e.g., Epic^{®} sensor plates optionally including a micro-fluidic component.

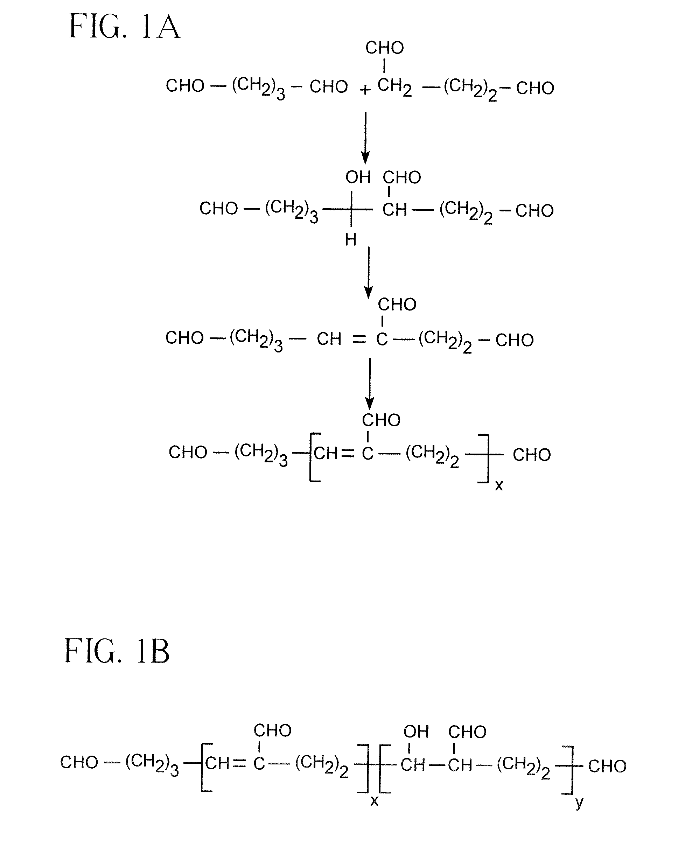

Referring to the Figures, Fig. 1A shows a schematic synthesis of polyglutaraldehyde (PGL) and Fig. 1B shows a possible alternative structure of a PGL polymer having both saturated and unsaturated aldehyde groups. Generally, the higher the value of "x" in formula of Fig. 1A (that is an alpha,beta-unsaturated aldehyde subunit or "mer"), the better the protein attachment results.

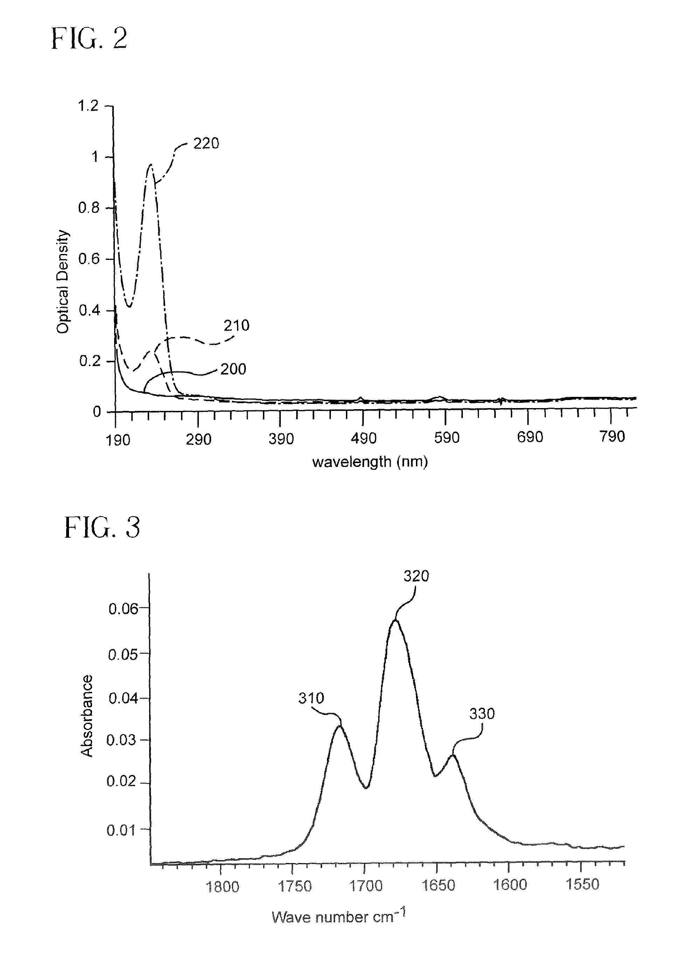

Fig. 2 shows the UV spectrum (optical density v. wavelength(nm)) of a water soluble polyglutaraldehyde of Example 1 at two concentrations by weight in water, 0.008% (210) and 0.0025% (220), compared to a monomeric glutaraldehyde at 0.008% (200). The UV spectrum was obtained using an HP UV spectrophotometer and a 1 cm optical pathway cuvette. The PGL crude solution from Example 1 was diluted 1,000 times with water.

Fig. 3 shows an exemplary FTIR spectrum of a water insoluble polyglutaraldehyde of Example 2 having both saturated and unsaturated aldehyde groups. The FTIR spectrum was recorded using a Nicolet Avatar FTIR spectrophotometer with the ATR device. Peaks were assigned as follows: unconjugated aldehydes (310), conjugated aldehydes (320), and conjugated carbon-carbon double bonds (C=C)(330).

Fig. 4 shows Cy5 labeled streptavidin (Cy5-SA) immobilization capacity at three pH values (from left to right: pH 5.5, 7.4, and 9, respectively) on an exemplary PGL surface (420) and compared with two reference or comparative aldehyde surfaces known as Superaldehyde 2 (400) (available from TeleChem International, Inc., www.arrayit.com) and glutaraldehyde activated surface (410) prepared according to U.S. patent application publication US2003/0092075A1, paragraph [0246], entitled "Protocol for Activating Amine-Coated Biosensor with Aldehyde." However, the patent publication does not mention the use of nanoparticles and does not mention the use of polyaldehyde nanoparticles. Although not limited by theory these differences are believed to be wholly- or partially-responsible for the superior immobilization capacity of the article, apparatus, and methods of the present disclosure. Protein concentration used in typical immobilization examples was, for example, about 100 micrograms/mL. Color fluorescence images (not included) of the abovementioned treated surfaces (400-420) appear to support and are consistent with the superior and unexpected immobilization capacity results illustrated in the Fig. 4 bar chart for the polyglutaraldehyde nanoparticulate treated surface (420) at each of the three pH values.

Fig. 5 shows a SEM micrograph of a deposited PGL colloid that was obtained from a water soluble PGL polymer. The PGL colloid was prepared by precipitation in pure water.

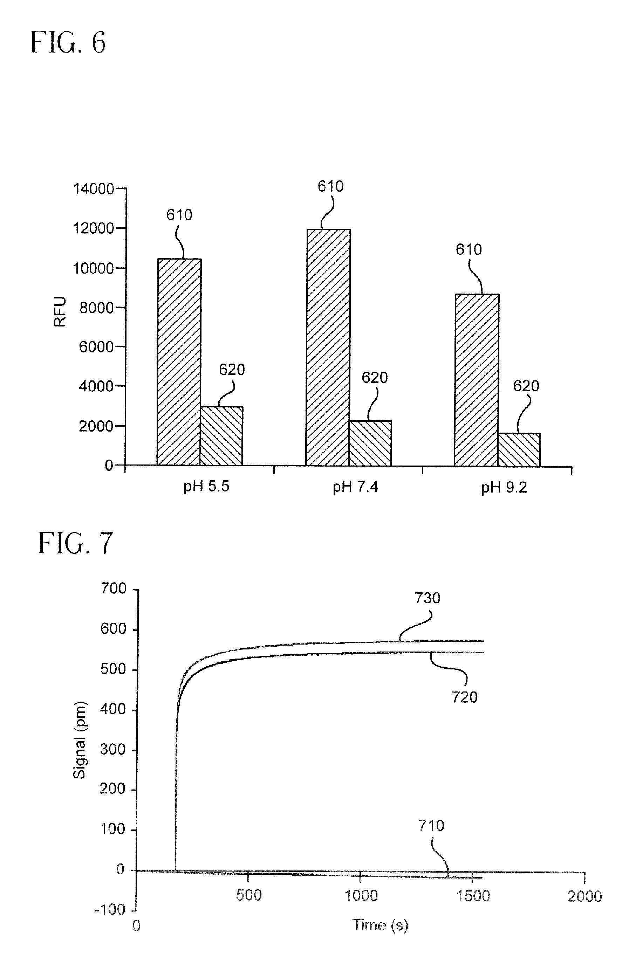

Fig. 6 shows exemplary values that demonstrate the level of Cy5-SA immobilization on a PGL coating obtained with a weak solvent (610) and a strong solvent mixture (620), respectively. A first PGL coating of Example 3A having a surface having PGL colloidal particles was prepared from a weak solvent (water). A second PGL coating having a uniform PGL coating and that was substantially free of PGL colloidal particles of Example 3B, was prepared from a strong solvent mixture (i.e., DMSO/H₂O:50/50:v/v). The samples were each prepared by first coupling the PGL particles on an aminopropylsilsesquioxane (APS) coated glass slide for 30 minutes. Next, Cy5-SA was immobilized on the PGL treated surfaces for 1 hr at 100 micrograms/mL at three different pH values, 5.5, 7.4, and 9, respectively, for both the weak (610) and the strong solvent (620) samples. The controls were surface chemistry (PGL coatings from weak or strong solvents) and without immobilization of the Cy5-SA. The controls had almost no observed fluorescence (e.g., less than about 500 RFU; which appeared as dark areas on well photos (not shown)).

Although not limited by theory, the improved immobilization levels observed when the PGL coating was prepared from a weak solvent or weak solvent mixture is believed to be due to the increased surface area provided by the nanoparticles presentation; more surface area permits more proteins to be adsorbed. In contrast, coating prepared from strong solvent provided coatings that were smooth and which surface coatings had lower surface area and lower protein absorbed.

Fig. 7 shows a Cy5-SA immobilization without NaCNBH₃ of Example 7A on a PGL coated Epic^{®} plate prepared with an exemplary PGL formulation of Example 4B at a fixed pH of 5.5 and three PGL concentration. The constant pH and concentration for the immobilization plot was as follows: pH 5.5 at 0 micrograms/mL (710), pH 5.5 at 25 micrograms/mL (720), and pH 5.5 at 50 micrograms/mL (730).

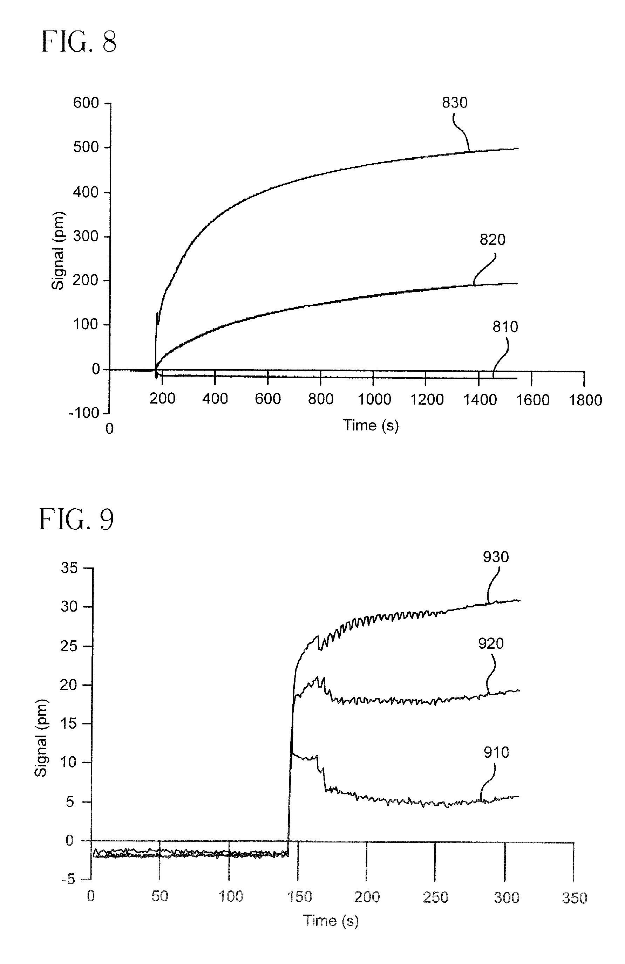

Fig. 8 shows an SA immobilization with NaCNBH₃ of Example 7B on a PGL coated Epic^{®} plate prepared with an exemplary PGL formulation of Example 4C. The constant pH and concentration for the immobilization plot was as follows: pH 5.5 at 0 micrograms/mL (810), pH 5.5 at 25 micrograms/mL (820), and pH 5.5 at 50 micrograms/mL (830).

Fig. 9 shows binding results of 4-fluorescein-biotin as described in Example 8, on streptavidin immobilized using NaCNBH₃ according to Example 7B, on a PGL Epic^{®} coated plate prepared according to Example 6, with a PGL formulation of Example 4C. The constant pH and concentration for the immobilization plot was as follows: pH 5.5 at 0 micrograms/mL (910), pH 5.5 at 25 micrograms/mL (920), and pH 5.5 at 50 micrograms/mL (930).

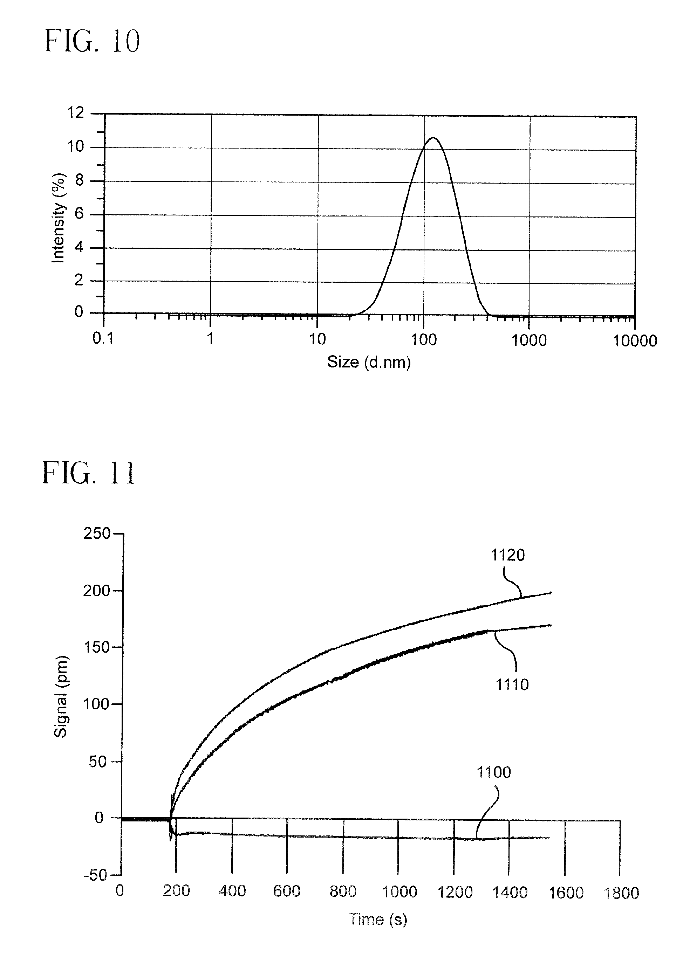

Fig. 10 shows tabulated and graphical average particle size and size distribution data as determined by dynamic light scattering (DLS) for a PGL nanoparticle dispersion obtained from precipitation from a 0.25% wt PGL in DMSO/H₂O-0.1% sodium dodecylsulfate (SDS) surfactant mixture from example 4C. The Z-Average particle size (d.nm) was 99.4, the Pdl was 0.212, and the intercept was 0.937. The accompanying Table 1 show some exemplary particle size results.

**Table 1. PGL particle size results.**

| **Peak No.** | **Particle Diameter (nm)** | **% Intensity** | **Width (nm)** |
|---|---|---|---|
| 1 | 128 | 100 | 64.5 |

Fig. 11 shows an exemplary SA immobilization using NaCNBH₃ according to Example 7B on an Epic^{®} PGL coated plate as described in Example 6 using the PGL formulation of Example 4A. The constant pH and concentration for the immobilization plot was as follows: pH 5.5 at 0 micrograms/mL (1100), pH 5.5 at 25 micrograms/mL (1110), and pH 5.5 at 50 micrograms/mL (1120).

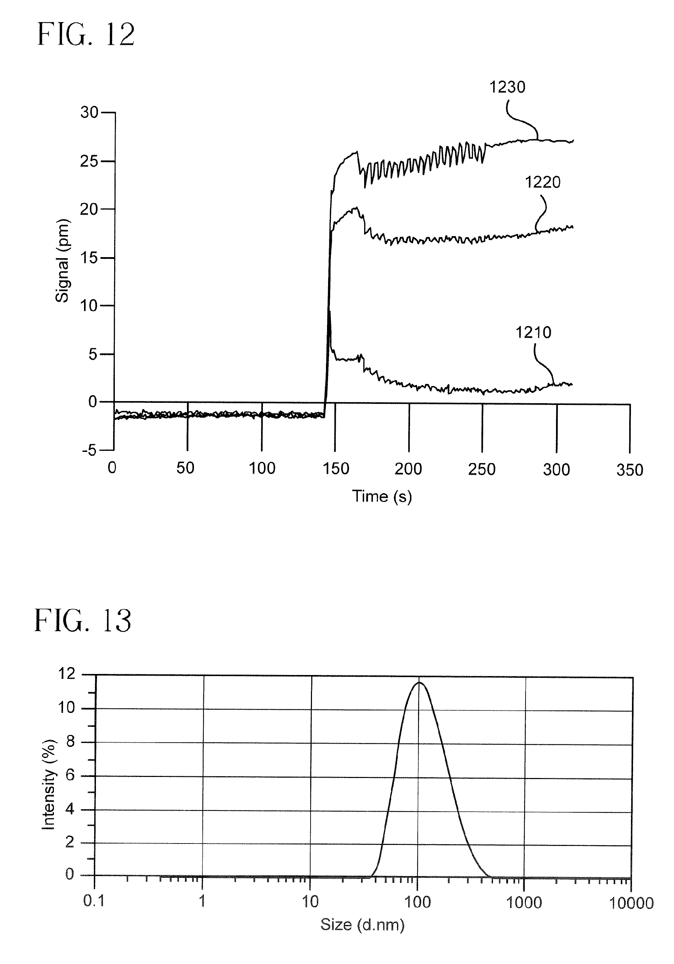

Fig. 12 shows binding of 4-fluorescein-biotin on streptavidin immobilized using NaBH₃CN accordingly to Example 8 on PGL Epic^{®} coated plate prepared using the PGL formulation of Example 4A. The constant pH and concentration for the immobilization plot was as follows: pH 5.5 at 0 micrograms/mL (1210), pH 5.5 at 25 micrograms/mL (1220), and pH 5.5 at 50 micrograms/mL (1230).

Fig. 13 shows average particle size data as determined by dynamic light scattering (DLS) for a precipitated PGL nanoparticle dispersion obtained from a 0.05% wt PGL in DMSO/H₂O-0.05% SDS mixture of Example 4A. The Z-Average particle size (d.nm) was 105, the Pdl was 0.175, and the intercept was 0.956. The accompanying Table 2 show exemplary particle size results.

**Table 2. PGL particle size results.**

| **Peak Number** | **Particle Diameter (nm)** | **% Intensity** | **Width (nm)** |
|---|---|---|---|
| 1 | 128 | 100 | 65.7 |

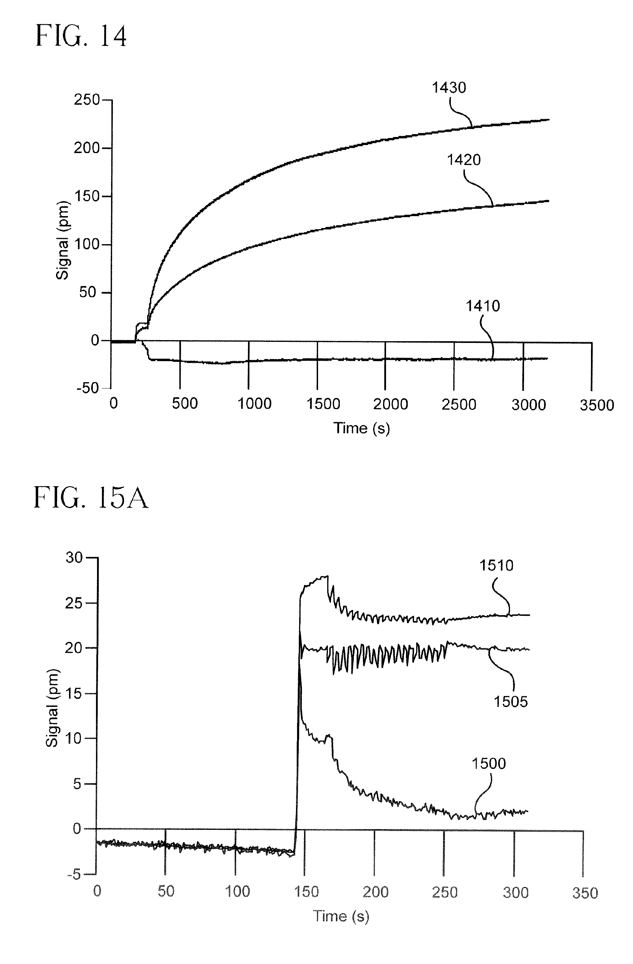

Fig. 14 shows exemplary SA immobilization without NaCNBH₃ accordingly to Example 9 on a PGL coated plate that was prepared as described in Example 6 using a PGL formulation of Example 4A. The constant pH and concentration for the immobilization plot was as follows: pH 5.5 at 0 micrograms/mL (1410), pH 5.5 at 25 micrograms/mL (1420), pH 5.5 at 50 micrograms/mL (1430).

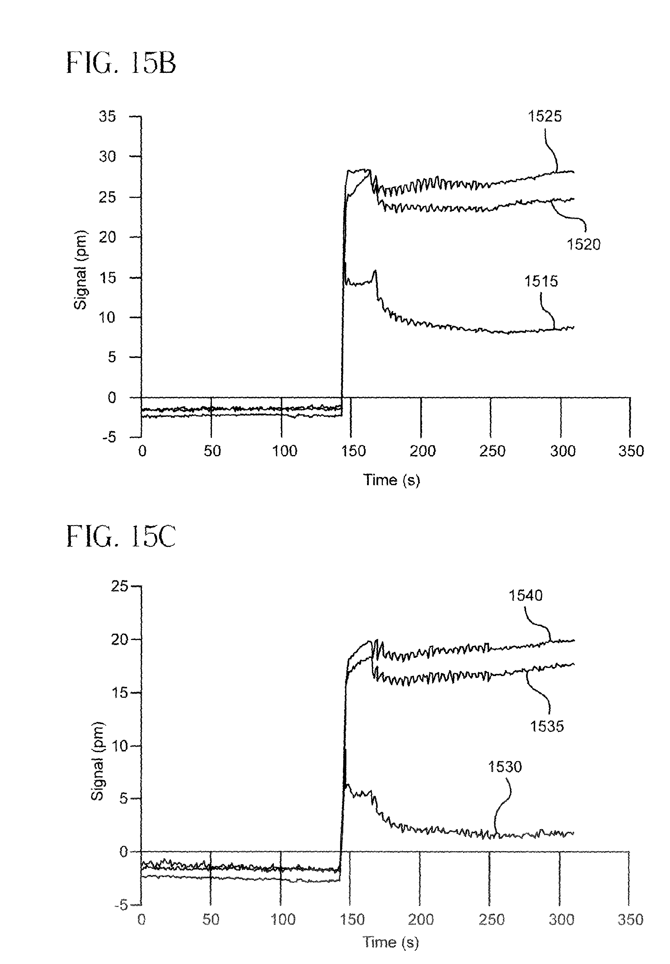

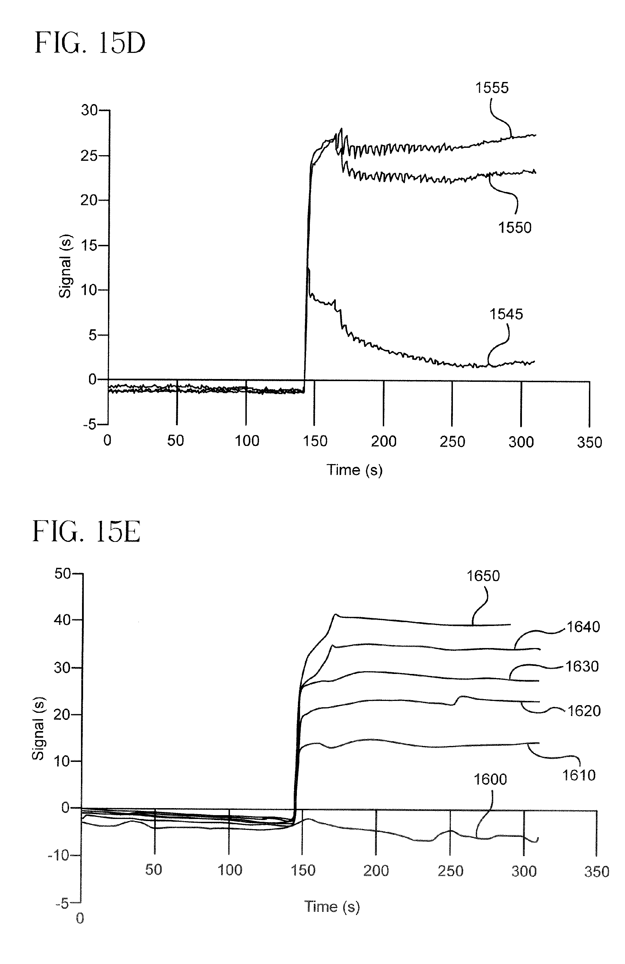

Fig. 15 shows exemplary binding results for experiments of 4-fluorescein-biotin on SA immobilized without NaCNBH₃ on a PGL coated Epic^{®} plate prepared using the formulations of Examples 4A, 4B, and 4C, respectively. The constant pH and concentration for the immobilization plots were as follows:
Fig. 15A: pH 5.5 at 0 micrograms/mL (1500), pH 5.5 at 25 micrograms/mL (1505), pH 5.5 at 50 micrograms/mL (1510);
Fig. 15B: pH 5.5 at 0 micrograms/mL (1515), pH 5.5 at 25 micrograms/mL (1520), pH 5.5 at 50 micrograms/mL (1525);
Fig. 15C: pH 5.5 at 0 micrograms/mL (1530), pH 5.5 at 25 micrograms/mL (1535), pH 5.5 at 50 micrograms/mL (1540); and
Fig. 15D: pH 5.5 at 0 micrograms/mL (1545), pH 5.5 at 25 micrograms/mL (1550), pH 5.5 at 50 micrograms/mL (1555).

Fig. 15E shows exemplary binding results of 4-fluorescein-biotin on streptavidin immobilized with K₂HPO₄ according to Example 10 on a PGL Epic^{®} coated plate prepared using a PGL formulation of Example 4A. The constant pH and concentration for the immobilization plot was as follows: pH 5.5 at 0 micrograms/mL (1600), pH 5.5 at 1.5 micrograms/mL (1610), pH 5.5 at 5 micrograms/mL (1620), pH 5.5 at 10 micrograms/ml (1630), pH 5.5 at 25 micrograms/mL (1640), and pH 5.5 at 50 micrograms/mL (1650).

### EXAMPLES

The following examples serve to more fully describe the manner of using the above-described disclosure, as well as to set forth the best modes contemplated for carrying out various aspects of the disclosure. It is understood that these examples in no way serve to limit the scope of this disclosure, but rather are presented for illustrative purposes.

### EXAMPLE 1

**Preparation of low molecular weight polyglutaraldehyde (PGL)** Materials: A 25 wt% solution of high purity grade glutaraldehyde from Sigma-Aldrich ref. G5882; 1 M aqueous sodium hydroxide; and 1M aqueous hydrochloric (HCl) acid solution.

A water soluble PGL was prepared according to US 6,326,136. 1M NaOH was added to a 25 wt% glutaraldehyde solution until the pH was 10. This solution was stirred at about 25 °C for 4 hours and then 1M HCl was added to lower the pH to about 4 to stop the polymerization reaction. The solution was clear without any visible precipitate.

### EXAMPLE 2

**Preparation of high molecular weight PGL** Materials: a 25 wt% solution of water insoluble PGL was prepared according to US 4,267,234; and 1 M aqueous sodium hydroxide solution. The 1M NaOH was added to the 25 wt% glutaraldehyde solution until the pH was 11.5. The solution was stirred for 2 hours at room temperature and precipitation of the water insoluble polyglutaraldehyde occurred after several minutes. At the end of two hours, the solution was heated at about 50°C for about 2 hrs and then the insoluble polyglutaraldehyde was isolated by centrifugation and washed thoroughly with deionized (DI) water. The isolated solid polyglutaraldehyde was dried by lyophilization for 4 hours at -80 °C under vacuum.

### EXAMPLE 3A

**Preparation of high molecular weight PGL** Materials: DI water; a solution containing the water soluble PGL of Example 1; and sodium cyanoborohydride (NaCNBH₃). About 9 gm of aqueous solution containing 0.1 wt% of NaCNBH₃ was added to 1 gm of a solution containing the water soluble PGL from Example 1. The solution was slightly hazy and contained about 2.5 wt % of PGL.

### EXAMPLE 3B

**Preparation of a solution without colloids from water soluble PGL in a DMSO:H₂O (1:1) mixture** Materials: DI water; DMSO; a solution containing the water soluble PGL of Example 1; and sodium cyanoborohydride (NaCNBH₃). A mixture of 4.5 gm DMSO and 4.5 gm of H₂O containing 0.1 w% of NaCNBH₃ was added to 1 gm of a solution containing the water soluble PGL of Example 1. The solution was clear and contained 2.5 wt% of PGL.

### EXAMPLE 4A-4E

**Preparation of a colloidal formulations from water insoluble PGL** Materials: dimethyl sulfoxide (DMSO); sodium dodecylsulfate (SDS) surfactant; sodium cyanoborohydride (NaCNBH₃); and water insoluble PGL powder from Example 2.

**EXAMPLE 4A** The water insoluble PGL powder from Example 2 was dissolved in DMSO to prepare a 1 wt % DMSO solution. Then to a solution of 13.3 gm of DI water containing 0.05 wt % SDS and 0.1 wt % NaCNBH₃ was added dropwise with vigorous stirring to 0.7 gm of the 1 wt% PGL/DMSO solution. The solution was slightly hazy and contained 0.05 wt % of PGL.

**EXAMPLE 4B** Water insoluble PGL powder from Example 2 was dissolved in DMSO to prepare a 1 wt % solution as in Example 4A. Then to a solution of 13.3 g of DI water containing 0.1 wt % SDS and 0.1 wt% NaCNBH₃ was added dropwise under vigorous stirring 0.7 gm of the 1wt % PGL/DMSO solution. The solution was slightly hazy and contained 0.05 wt% of PGL.

**EXAMPLE 4C** The Example 4B procedure was repeated except that a 5 wt% PGL formulation in DMSO was used instead of a 1 wt% solution. The solution appeared slightly hazy and contained 0.25 wt % of PGL.

**EXAMPLE 4D** The Example 4B procedure was repeated except that a 10 wt % PGL formulation in DMSO was used instead of a 1 wt % solution. The solution appeared slightly hazy and contained 0.5 wt % of PGL.

**EXAMPLE 4E** The Example 4A procedure was repeated except that a 10 wt % PGL formulation in DMSO was used instead of a 1 wt % solution. The solution appeared slightly hazy and contained 0.5 wt % of PGL

### EXAMPLE 5

**Grafting of PGL colloids prepared from a water soluble PGL onto aminated glass slides and fluorescence experiments** Materials: DI water; Corning^{®} 1737 clean glass slides; aminopropylsilane (APS) 20 wt % solution in water from ABCR; and the PGL colloid of Example 3.

The Coming^{®} 1737 pyrolyzed glass slides were soaked for 30 min in a 1 wt % APS solution. Then the slides were washed extensively with water followed by extensive rinsing with isopropyl alcohol (IPA) and finally dried in a gentle argon stream. The dried slides were immersed in the PGL formulation for 30 min. Then the slides were rinsed extensively with water and dried under a gentle argon stream.

### EXAMPLE 6

**Preparation of PGL colloids coated Epic^{®} plate** Materials: DI water; Coming Epic^{®} 96-well plate; APS 20 wt % solution in water from ABCR; the PGL colloids from Examples 4A to 4E, and sodium cyanoborohydride.

A 1 wt % solution of APS in DI water was prepared from the 20 wt% commercially available solution. 75 microliters of the diluted solution were added in each well and then incubated for 10 min under gentle shaking. After incubation, the APS solution was removed, the plate was rinsed extensively with water and with ethyl alcohol, and then dried with a gentle stream of nitrogen. After drying, 100 microliters of the PGL colloid solution from Example 4 was added into each well and incubated for 2 hrs at room temperature. Then excess solution was removed and the plate was rinsed three times with water. After rinsing, the plate was dried by with a gentle argon stream. The resulting LID plate was ready for LID assay without any further activation.

### EXAMPLE 7A

### Cy5 -SA immobilization on PGL Epic^{®} plate without cyanoborohydride

Materials: Cy5-labeled Streptavidine is a fluorescent labeled protein from Amersham Bioscience Europe; sodium acetate buffer; PGL coated Epic^{®} plates from Example 6. 75 microliters of sodium acetate buffer (pH 5.5) was dispensed into each well. Then alignment of the optical parts was performed and the plate was left undisturbed until reaching a stable baseline. After the stable baseline (i.e., no visible response drift) was reached, 75 microliters of a solution containing 0, 50, or 100 micrograms/mL of Cy5-SA in acetate buffer was introduced in the wells and mixed for 20 minutes using a fill/aspirate protocol. The immobilization response was recorded during these 20 minutes. This example demonstrated that Cy5-SA binds to the PGL coating without the addition of cyanoborohydride.

### EXAMPLE 7B

### SA immobilization on PGL Epic^{®} plate with sodium cyanoborohydride

Materials: Streptavidine (un-labeled) ref. S4762 from Sigma-Aldrich; and sodium cyanoborohydride. The same procedure as Example 7A was used except that the Cy5- SA was replaced by an unlabeled-SA and the 0.1 wt% of sodium cyanoborohydride was added in the buffer. This example demonstrated that SA bind significantly to the PGL coating with cyanoborohydride.

### EXAMPLE 8

### fl-Biotin binding on SA previously immobilized on a PGL coated Epic^{®} plate

Materials: Fluorescein-Biotin from Sigma-Aldrich ref. B9431; 0.01 M PBS (phosphate buffer); 0.0027 M potassium chloride; and 0.137 M sodium chloride pH 7.4; and dimethylsulfoxide (DMSO).

Preparation of 800 nM fl-biotin solution: 16 microliters of 500 µM fl-biotin solution in DMSO were added to 10 mL of PBS and mixed.

Preparation of PBS/DMSO solution: 16 microliters of DMSO were added to 10 mL of PBS.

Each well of the Epic^{®} plate from Examples 7A, 7B, 9, and 10 was rinsed three (3) times with water using an automatic pipettor. Then 75 microliters of the PBS/DMSO solution were added to each well and the alignment of the optical parts was performed. The plate was left undisturbed until reaching a stable baseline. After reaching a stable base line, 75 microliters of the fl-biotin solution were added to each well and mixed continuously for 2 mins using an automatic pipettor. In the course of the mixing, the binding curve was recorded in real time.

### EXAMPLE 9

### SA immobilization on a PGL Epic^{®} plate without cyanoborohydride

Streptavidine was immobilized on PGL Epic^{®} coated plates as described in Example 7A except that unlabelled SA was used instead of Cy5-SA and mixing was performed for 50 min. instead of 20 min.

### EXAMPLE 10

### SA immobilization on PGL Epic^{®} plate using 2M KH₂PO₄ and fl-biotin binding

Materials: Unlabeled Streptavidine; KH₂PO₄ from Sigma-Aldrich ref. P5504; and PBS buffer. First, a 4 M KH₂PO₄ solution was prepared and its pH was adjusted to 8. Then 75 microliters of this solution were added to each well from a PGL Epic^{®} coated plate prepared using PGL formulation of Example 4A. The alignment of the optical parts was performed and the plate was left undisturbed until a stable baseline was reached. After reaching a stable baseline, 75 microliters of PBS solution containing 100, 50, 20, 10, 3, or 0 micrograms/mL SA were added in their respective wells and mixed for 1 hour. The final SA concentrations were 50, 25, 10, 5, 1.5, and 0 micrograms/mL SA, respectively. After rinsing, the fl-biotin binding was performed as described in Example 8. This example shows that binding of fl-biotin can be easily observed even when the protein was immobilized at a very low concentration such as 1.5 micrograms/mL. This example also demonstrates the low protein consumption provided in embodiments of the disclosure.

The disclosure has been described with reference to various specific embodiments and techniques. However, it should be understood that many variations and modifications are possible while remaining within the spirit and scope of the disclosure.

### References

1. Greg T. Hermanson, "Bioconjugate Techniques" p.146, Academic Press Inc. 1996.
2. Isosaki K, et al., "Immobilization of Proteins Ligands with Methyl Vinyl Ether Maleic-Anhydride Copolymers," J. of Chromatography, . 1992, April 24; 597 p 123-128.
3. Satoh A, et al., "Immobilisation of saccharides and peptides on 96-well microtiter plates coated with vinyl ether maleic anhydride copolymer," Anal. Biochemistry, June 1998, Vol. 260, no. 1, p. 96-102.
4. JP 05271299 "Protein A-Immobilized adsorbent."
5. US Patent Application Publication 2006/0110594, USSN 10/996,953, to Frutos, et al., entitled "Polymer-Coated Substrates for the Binding of Biomolecules and Methods of Making and Using Thereof."
6. US Patent Application Publication 20070154348, USSN 60/754,747, filed Dec. 29, 2005, to Frutos, et al., entitled "Supports for Assaying Analytes and Methods of Making and Using Thereof."
7. Greg T. Hermanson, et al., p.69, "Immobilized Affinity Ligand Techniques," Academic Press Inc., 1992.
8. A. Rembaum, et al., "Synthesis and Characterization of Poly(glutaraldehyde). A potential reagent for protein immobilization and cell separation," Macromolecules, 1980, 13, 19-24.
9. Jayakrishnan, A., "Glutaraldehyde as a fixative in bioprostheses and drug delivery matrices," Biomaterials, 1996, Vo1.17, 5:471-484; Rembaum, A., et al. "Reaction of polyglutaraldehyde with proteins," Polym. Prep Am. Chem. Soc Div Polym., 1978; 19: 648-650.
10. US 6,326,136 B1, to Lazar, et al., entitled "Macromolecular conjugate made using unsaturated aldehydes."
11. US 4,369,226, to Rembaum, entitled "polyglutaraldehyde synthesis and protein bonding substrates."
12. Wein chen, et al., Langmuir, 2006, 22, 8192-8196.
13. US 6,733,894 B2, to Ho, et al., entitled "High density functional slide and preparation method thereof."
14. US 5,543,456, to Iriguchi, et al., entitled "Process for preparing an aqueous resin dispersion and an aqueous resin dispersion obtained by the process."

## Claims

1. A method of making an article, the method comprising:
providing a functionalized substrate;
contacting the functionalized substrate with a polyglutaraldehyde nanoparticulate formulation to deposit polyglutaraldehyde nanoparticles on the functionalized substrate.

2. The method of claim 1 wherein the functionalized substrate comprises a glass sheet having a surface treated with an amino-hydrocarbylsilane, a hydrazido-hydrocarbylsilane, an amino-hydrocarbyl silsesquioxane, a hydrazido-hydrocarbyl silsesquioxane, or combinations thereof.

3. The method of claim 1 or 2 wherein the polyglutaraldehyde nanoparticulate formulation comprises polyglutaraldehyde nanoparticles in a mixture of H₂O:DMSO in a volume ratio of from about 100:0 to about 80:20.

4. The method of claim 1 or 2 wherein the polyglutaraldehyde nanoparticulate formulation comprises from about 1 to about 5 wt% polyglutaraldehyde nanoparticles suspended in H₂O.

5. The method of claim 1 or 2 wherein the polyglutaraldehyde nanoparticulate formulation comprises polyglutaraldehyde nanoparticles, a liquid comprised of H₂O, or a mixture of H₂O and DMSO, a surfactant, and a water soluble base.

6. The method of any one of claims 1 to 5 wherein the polyglutaraldehyde nanoparticulate formulation comprises a reducing agent in an amount of from about 0.01 wt% to about 1 wt%.

7. The method of claim 6 wherein the reducing agent comprises NaCNBH₃.

8. An article, more particularly suitable for label-independent detection, the article comprising:
a substrate comprising a contact surface comprising a tie layer having polyglutaraldehyde nanoparticles deposited on the tie layer.

9. The article of claim 8 wherein the substrate comprises at least one of a glass, a polymer, a composite, or combinations thereof.

10. The article of claim 8 or 9 wherein the polyglutaraldehyde nanoparticles have a surface area coverage on the article of from about 0.01 % to about 10%.

11. The article of any one of claims 8 to 10 wherein the polyglutaraldehyde nanoparticles have a particle diameter of from about 1 to about 1,000 nanometers.

12. The article of any one of claims 8 to 11 wherein the polyglutaraldehyde nanoparticles have a particle diameter of from about 50 to about 150 nanometers.

13. The article of any one of claims 8 to 12 wherein the polyglutaraldehyde nanoparticles comprise a polyglutaraldehyde having at least one of a saturated aldehyde, an unsaturated aldehyde, or mixtures thereof.

14. An article, more particularly suitable for label-independent detection, the article comprising:
a substrate comprising a contact surface comprising polyglutaraldehyde nanoparticles.

15. The article of claim 14 wherein the nanoparticles are covalently bonded to the substrate.

16. The article of claim 14 wherein the nanoparticles are deposited on the substrate.

17. The article of any one of claims 14 to 16 wherein the substrate comprises at least one of: an aminated plastic, an aminated glass, an aminated composite, or combinations thereof.

18. A method of immobilizing a biomolecule, the method comprising:
contacting the contact surface of an article of any one of claim 8 to 17 with a biomolecule; and
optionally rinsing and drying the contacted surface.

19. The method of claim 18 wherein the biomolecule comprises at least a protein, a nucleic acid, a pathogen, a cell structural component, a cell, or combinations thereof.

20. An apparatus for label-independent detection, the apparatus comprising an optical biosensor having a contact surface comprising an article according to any one of claims 8 to 17.
